# EUROPÄISCHE PATENTSCHRIFT

(11) **EP 3 487 433 B1**
(45) Veröffentlichungstag und Bekanntmachung des Hinweises auf die Patenterteilung: **26.08.2020**
(21) Anmeldenummer: 18745924.3
(22) Anmeldetag: 25.07.2018
(51) Int. Cl.: A61B 17/86, A61B 17/70, A61B 17/68

(54) **KNOCHENANKER FÜR TRIANGULÄRE ILIOSAKRALOSTHEOSYNTHESE**
BONE ANCHOR FOR TRIANGULAR ILIOSACRAL OSTEOSYNTHESIS
ANCRAGE OSSEUX POUR OSTÉOSYNTHÈSE SACRO-ILIAQUE

(30) Priorität: 25.07.2017 DE 102017116755
(43) Veröffentlichungstag der Anmeldung: 29.05.2019
(73) Patentinhaber: Silony Medical International AG, 8500 Frauenfeld (CH)
(72) Erfinder: VORDEMVENNE, Thomas, 48143 Muenster (DE); JOSTEN, Christoph, 04109 Leipzig (DE); KANDZIORA, Frank, 63322 Rödermark (DE)
(74) Vertreter: DREISS Patentanwälte PartG mbB
(86) Internationale Anmeldenummer: PCT/EP2018/070162
(87) Internationale Veröffentlichungsnummer: WO 2019/020690

(56) Entgegenhaltungen:
- WO-A1-2013/000071
- WO-A2-2011/014135
- US-A1- 2015 190 187

## Beschreibung

Die Erfindung betrifft einen Knochenanker für trianguläre Iliosakralostheosynthese mit einer Iliumschraube mit einer ersten Längsrichtung und einer Iliumsakralschraube mit einer zweiten Längsrichtung.

Aus US 2014/0135850 A1 und US 2015/0190187 A1 ist ein solcher Knochenanker bekannt, wobei die Iliumschraube durch eine langlochförmige Öffnung in der Iliumsakralschraube hindurchgeführt ist. Dies bedeutet aber eine praktische Limitierung für den Außendurchmesser der Iliumschraube bzw. die Iliumsakralschraube muss mit möglicherweise überdimensioniert großem Durchmesser ausgeführt werden, was wiederum Nachteile mit sich bringt. Einige Figuren zeigen eine Ausführungsform, bei der die Iliumschraube in einem gewindelosen Abschnitt mit einer langlochförmigen Öffnung ausgebildet ist, durch welche die Iliumsakralschraube hindurchgeführt und in das Sakrum einschraubbar ist.

Der vorliegenden Erfindung liegt die Aufgabe zugrunde, einen Knochenanker für trianguläre Iliosakralostheosynthese zu schaffen, bei dem eine Fraktur des Os Sacrum auf verbesserte Weise behandelt werden kann, was auch eine günstige Implantierbarkeit des Knochenankers einschließt.

Diese Aufgabe wird erfindungsgemäß gelöst durch einen Knochenanker der eingangs genannten Art, wobei die Iliumschraube in einem gewindelosen Abschnitt eine vorzugsweise langlochförmige Durchgangsöffnung quer zur ersten Längsrichtung aufweist, wobei die Iliumsakralschraube einen solchen gegenüber der Iliumschraube kleineren Außendurchmesser aufweist, dass sie durch diese Durchgangsöffnung hindurchführbar ist und ausgehend vom Ilium durch die Iliumschraube hindurch in das Sacrum einschraubbar ist, wobei die Iliumschraube einen distalen Gewindeabschnitt aufweist mit einem distalen Kerndurchmesser und einem distalen Nenndurchmesser des Gewindes und einen proximalen Gewindeabschnitt mit einem proximalen Kerndurchmesser und einem proximalen Nenndurchmesser des Gewindes, und wobei der gewindelose Abschnitt zwischen dem distalen und dem proximalen Gewindeabschnitt angeordnet ist, wobei der proximale Nenndurchmesser größer ist als ein Außendurchmesser des gewindelosen Abschnitts und der Außendurchmesser des gewindelosen Abschnitts größer ist als der distale Nenndurchmesser, und wobei in die Durchgangsöffnung ein der Form der Durchgangsöffnung komplementär entsprechendes Hülsenelement aus einem Polymermaterial eingesetzt ist, welches in der Durchgangsöffnung dann reibschlüssig und zusätzlich bezüglich mindestens einer Richtung formschlüssig gehalten ist, wobei eine Innenabmessung des Hülsenelements orthogonal zur ersten Längsrichtung geringer ist als ein Nenndurchmesser eines Gewindes der Iliumsakralschraube.

Dadurch, dass die insbesondere langlochförmige Durchgangsöffnung in der Iliumschraube ausgebildet ist, also die Iliumsakralschraube durch diese Durchgangsöffnung in der Iliumschraube hindurchgeführt wird, ist es ohne weiteres möglich, die Iliumsakralschraube mit einem Durchmesser von lediglich 6 mm - maximal 9 mm auszubilden, was sich für die Anwendungen als vorteilhaft erweist. Dies bedeutet weiter, dass der Außendurchmesser der Iliumschraube zumindest im Bereich der Durchgangsöffnung so groß bemessen werden muss, damit die Durchgangsöffnung die erforderliche Größe für die Iliumsakralschraube aufweisen kann. Dies erweist sich als weniger problematisch, da die Iliumschraube in einem Bereich des Iliums angeordnet wird, wo die anatomische Ausprägung des Iliums und insbesondere die Knochenstärke des Iliums dies zulässt.

Des weiteren kann durch das in die Durchgangsöffnung eingesetzte Hülsenelement aus Polymermaterial eine gute und stabile Fixierung der Iliumsakralschraube bezüglich der Iliumschraube erreicht werden. Da weiter erfindungsgemäß das Hülsenelement im eingepressten Zustand nicht nur reibschlüssig, sondern zusätzlich zumindest in einer Richtung, das heißt, bezüglich der Einpressrichtung oder in der Gegenrichtung, formschlüssig in der Durchgangsöffnung gehalten ist, lässt sich sicher ausschließen, dass sich das Hülsenelement von seiner bestimmungsgemäßen Position in der Durchgangsöffnung löst, insbesondere beim Einschrauben der Iliumsakralschraube, wenn die Iliumsakralschraube mit ihrem Gewinde in das Material des Hülsenelements einschneidet. Sofern ein solcher formschlüssiger Hintergriff nur in einer Richtung realisiert ist, was ausreichend ist, so erweist es sich als vorteilhaft, wenn der formschlüssige Hintergriff bezüglich dieser einen Richtung so orientiert ist, dass beim Einschrauben der Iliumsakralschraube das Hülsenelement nicht in Löserichtung sondern in Richtung des Formschlusses belastet wird. Hierbei sollte der Chirurg also darauf achten, dass die Iliumschraube mit ihrem eingesetzten Hülsenelement in der entsprechenden Drehstellung orientiert ist, wenn die Iliumsakralschraube eingesetzt wird. Bei einem formschlüssigen Hintergriff in Einpressrichtung und entgegen der Einpressrichtung kommt es auf diese Orientierung hingegen nicht an.

Es erweist sich als vorteilhaft, wenn das Hülsenelement aus einem zwar formstabilen, jedoch geringfügig nachgiebig verformbaren, insbesondere elastisch nachgiebig verformbaren, Polymermaterial besteht, so dass es in Bezug auf die Durchgangsöffnung mit geringfügigem Übermaß ausgebildet und dann unter geringfügiger Verformung in die Durchgangsöffnung eingepresst werden kann.

Das Hülsenelement ist vorzugsweise aus Polyethylen, insbesondere aus ultra-hochmolekularem Polyethylen (UHMW PE), aus PEEK, PEAK, PEKK, PPSU oder PPS, gebildet. Bevorzugtermaßen wird nicht vernetztes Polyäthylen verwendet, welches nicht zur Brüchigkeit neigt.

Weiter erweist es sich als vorteilhaft, dass eine Innenabmessung der Durchgangsöffnung orthogonal zur ersten Längsrichtung wenigstens 6,0 mm, insbesondere wenigstens 7,0 mm und weiter insbesondere höchstens 11,5 mm, insbesondere höchstens 10,5 mm, insbesondere höchstens 9,5 mm beträgt.

Es wird weiter vorgeschlagen, dass eine Innenabmessung des Hülsenelements orthogonal zur ersten Längsrichtung geringer ist als ein Kerndurchmesser eines Gewindes der Iliumsakralschraube. Auf diese Weise kann eine zu starke Verdrängung oder plastische Verformung des Materials des Hülsenelements vermieden werden, indem lediglich die Gewindegänge der Iliumsakralschraube, nicht aber deren Kern in das Hülsenelement einschneiden oder dessen Material verdrängen.

Wie eingangs schon angedeutet, erweist es sich als vorteilhaft, dass ein Nenndurchmesser eines Gewindes der Iliumsakralschraube wenigstens 6 mm und höchstens 9,5 mm, insbesondere höchstens 8,5 mm beträgt.

Für die Ausbildung des Hülsenelements wird weiter vorgeschlagen, dass das Hülsenelement eine Wandstärke von wenigstens 1,0 mm, insbesondere von wenigstens 1,5 mm und von höchstens 3,5 mm, insbesondere von höchstens 3,0 mm, insbesondere von höchstens 2,5 mm aufweist.

Um den formschlüssig wirkenden Hintergriff bezüglich der Einpressrichtung oder möglicherweise bezüglich der Gegenrichtung zu realisieren, wäre es möglich, dass die Öffnung in der Einpressrichtung oder in der Gegenrichtung in geeigneter Weise sich verjüngend auszubilden, insbesondere konisch oder mit keilförmig zueinander erstreckten Begrenzungsflächen auszubilden, wobei das Hülsenelement vorzugsweise in komplementärer Weise auszubilden wäre. Nach einer weiteren Ausführungsform der Erfindung wird vorgeschlagen, dass das Hülsenelement wenigstens einen quer zu seiner Einpressrichtung vorstehenden Ansatz aufweist, mittels dessen ein formschlüssiger Hintergriff des Hülsenelements mit der Iliumschraube erreicht wird. Dieser Ansatz kann dann gegen eine insbesondere entsprechend komplementär ausgebildete Gegenlagerstelle an der Iliumschraube anlaufen, wodurch sich der formschlüssige Hintergriff realisieren lässt.

In weiterer Ausbildung dieses Gedankens erweist es sich als vorteilhaft, wenn der Ansatz eine Abstufung an dem Hülsenelement bildet. Dieser Ansatz oder diese Abstufung ist weiter vorzugsweise in einer Umfangsrichtung des Hülsenelements erstreckt. Der Ansatz oder die Abstufung können dabei einen in der Umfangsrichtung unterbrochenen oder in der Umfangsrichtung durchgehenden Flansch an dem Hülsenelement bilden. Bei dieser Ausführung kann das Hülsenelement über seinen Umfang gleichmäßig im eingesetzten Zustand gestützt werden, wodurch der formschlüssige Hintergriff dann ebenfalls gleichmäßig das Hülsenelement über dessen Umfang stützt, sofern Kräfte auf das Hülsenelement eingeleitet werden, wie insbesondere beim Einschrauben der Iliumsakralschraube.

Des Weiteren erweist es sich als vorteilhaft, dass die Durchgangsöffnung mit einer Ausnehmung oder Abstufung ausgebildet ist, welche beim Einpressen des Hülsenelements einen Hintergriff mit dem Hülsenelement ausbildet oder einen Endanschlag für das Hülsenelement, insbesondere für dessen Ansatz oder Abstufung, bildet. Hierbei wäre es natürlich auch denkbar, dass die Durchgangsöffnung mehrere Ausnehmungen oder Abstufungen aufweist. Insbesondere wäre es denkbar, dass die Ausnehmung eine in der Umfangsrichtung erstreckte Nut aufweist, in welche das Hülsenelement mit Ansätzen oder sonstigen Eingriffsbereichen formschlüssig eingreifen kann. Demgegenüber ist die Ausbildung einer Abstufung einfach herstellbar und das Hülsenelement lässt sich, vorzugsweise mit einer entsprechend konfigurierten Abstufung in der Einpressrichtung, problemlos bis auf Anschlag in die korrekte Montageposition bringen.

Weiter erweist es sich als vorteilhaft, dass die Iliumschraube kanüliert ausgebildet ist und dass das Hülsenelement eine mit der ersten Längsrichtung und somit mit der Kanülierung fluchtende Durchgangsöffnung aufweist. Auf diese Weise kann auch die Iliumschraube mit eingesetztem Hülsenelement über einen Führungsdraht bei der Implantation zugeführt werden.

Es wird weiter vorgeschlagen, dass der distale Nenndurchmesser des distalen Gewindeabschnitts der Iliumschraube wenigstens 5,0 mm, insbesondere wenigstens 6,0 mm, insbesondere wenigstens 7,0 mm und höchstens 10,5 mm, insbesondere höchstens 9,5 mm, insbesondere höchstens 8,5 mm beträgt.

Weiter wird vorgeschlagen, dass der Außendurchmesser des gewindelosen Abschnitts im Bereich der Durchgangsöffnung wenigstens 9,0 mm, insbesondere wenigstens 10,0 mm und höchstens 14,5 mm, insbesondere höchstens 13,5 mm, insbesondere höchstens 12,5 mm beträgt.

Weiter wird vorgeschlagen, dass der proximale Nenndurchmesser des proximalen Gewindeabschnitts der Iliumschraube wenigstens 10,0 mm, insbesondere wenigstens 11 mm und höchstens 16,5 mm, insbesondere höchstens 15,5 mm, insbesondere höchstens 14,5 mm beträgt.

Nach einer weiteren Ausführungsform der Erfindung erweist es sich als besonders vorteilhaft, dass ein Außendurchmesser der Iliumschraube ausgehend von dem gewindelosen Abschnitt und von einer Stelle distal der Durchgangsöffnung in distaler Richtung bis zu dem distalen Gewindeabschnitt abnimmt und insbesondere in den distalen Kerndurchmesser des distalen Gewindeabschnitts ausläuft. Durch dieses weitere Konfigurationsmerkmal ist die Iliumschraube in einem distalen Bereich, der sich in der Längsrichtung insbesondere bis zur Mitte der Iliumschraube erstrecken kann, mit einem geringeren Außendurchmesser ausgebildet. Dies erweist sich als vorteilhaft, da die Knochenstärke des Iliums in dem Bereich, wo die Iliumschraube distal zu liegen kommt, typischerweise geringer ist oder abnimmt.

Hierbei kann es sich weiter als vorteilhaft erweisen, dass ein Außendurchmesser der Iliumschraube ausgehend von dem gewindelosen Abschnitt und von einer Stelle distal der Durchgangsöffnung in distaler Richtung bis zu dem distalen Gewindeabschnitt abnimmt und dass in dem Bereich abnehmenden Außendurchmessers der Iliumschraube ein weiteres Außengewinde mit entsprechend abnehmendem Nenndurchmesser ausgebildet ist. Hierdurch kann der gewindelose Bereich der Iliumschraube, in dem die Durchgangsöffnung ausgebildet ist, über den distal angrenzenden Gewindeabschnitt noch stabiler stützend im Ilium verankert werden.

Der proximale Endbereich der Iliumschraube kann ohne besondere Einschränkungen gestaltet werden. Es kann sich aber als vorteilhaft erweisen, dass an einem proximalen Endbereich der Iliumschraube ein Kugelkopfabschnitt festlegbar, insbesondere in ein dort vorgesehenes Innengewinde einschraubbar, ist, wobei der
Kugelkopfabschnitt in einem weiteren gabelförmigen Aufnahmeteil in einer auswählbaren Orientierung festlegbar aufgenommen ist, wobei an dem Aufnahmeteil zudem ein Stab festlegbar ist, der über einen weiteren Knochenanker bezüglich eines Wirbelsäulensegments festlegbar ist. Dies eröffnet die Möglichkeit, dass der im Ilium und Sakrum festgelegte Knochenanker zusätzlich mit einem oder mehreren Wirbelsäulensegmenten starr gekoppelt werden kann, sofern sich dies aus medizinischer Sicht als ratsam erweist.

Es kann sich auch als vorteilhaft erweisen, dass an einem proximalen Endbereich der Iliumschraube eine Kappe festlegbar, insbesondere in ein dort vorgesehenes Innengewinde einschraubbar, ist.

Weiter kann es sich als vorteilhaft erweisen, dass der proximale Endbereich der Iliumschraube eine axiale Öffnung mit Innengewinde aufweist, in welche der Kugelkopfabschnitt oder die Kappe oder ein Werkzeug mit einem jeweiligen Außengewindeabschnitt einschraubbar ist.

Vorteilhafter Weise ist der proximale Endbereich der Iliumschraube mit einer Werkzeugansetzstelle ausgebildet, die es gestattet, eine Drehkopplung mit einem Werkzeug rasch herzustellen und wieder zu lösen.

Weiter erweist es sich als vorteilhaft, dass an einem proximalen Endbereich der Iliumschraube ein Werkzeug zum Eindrehen der Iliumschraube und/oder eine Zielschablone lösbar anbringbar ist, mittels derer ein Führungsdraht gezielt in einer vorwählbaren Orientierung bezüglich der Durchgangsöffnung in der Iliumschraube führbar und im Sakrum anordenbar ist.
Es erweist sich weiter als vorteilhaft, dass die Iliumschraube an einem proximalen Endbereich eine Werkzeugansetzstelle aufweist, welche eine die Einpressrichtung oder Einklipsrichtung des Hülsenelements und damit dessen Orientierung in Bezug auf einen Endanschlag in der Durchgangsöffnung bezeichnende Markierung aufweist.

Die Iliumsakralschraube ist vorzugsweise mit einem eine Werkzeugansetzstelle, zum Beispiel Inbus, Torx, aufweisenden Kopf ausgebildet, der an einen Schaft der Iliumsakralschraube anfügbar oder einstückig mit dem Schaft ausgebildet ist.

Weiter erweist es sich als vorteilhaft, dass die Iliumsakralschraube kanüliert ausgebildet ist. Auf diese Weise kann die Iliumsakralschraube über einen Führungsdraht bei der Implantation zugeführt werden.

Weiter erweist es sich als vorteilhaft, dass die Iliumsakralschraube im distalen Drittel mit seitlichen Perforierungen ausgebildet ist und somit ein marktüblicher Knochenzement durch die Schraube appliziert werden kann. Dadurch kann eine zusätzliche Fixierung der Iliumsakralschraube im sakralen Wirbelkörper erreicht werden.

Im eingeschraubten Zustand kann die Iliumsakralschraube dann mit ihrem Kopf an die Außenseite des Iliums anliegen. Nach einem weiteren Erfindungsgedanken wird demgegenüber vorgeschlagen, dass die Iliumsakralschraube eine Anlagescheibe für die Anlage an eine Iliumoberfläche aufweist, welche radial geschnitten und derart ausgebildet ist, dass sie auf einen eingeschnürten Bereich im Bereich eines proximalen Endes oder Kopfs der Iliumsakralschraube aufklipsbar ist und dort zur zweiten Längsrichtung neigbar jedoch unverlierbar angeordnet ist, so dass sie sich beim Festschrauben der Iliumsakralschraube an die Iliumoberfläche auch in einem Winkel zur zweiten Längsrichtung anlegen kann. Auf diese Weise kann die Iliumsakralschraube über ihre Anlagescheibe flächenhaft gegen eine Außenseite des Iliums anliegen, auch wenn diese Außenseite nicht orthogonal zur Längsrichtung der Iliumsakralschraube verläuft. Dies erweist sich als besonders vorteilhaft, da hierdurch eine weitere Stabilisierung der eingeschraubten Iliumsakralschraube erzielt werden kann.

In noch weitergehender Ausbildung dieses Gedankens wird vorgeschlagen, dass die Anlagescheibe und das proximale Ende oder der Kopf der Iliumsakralschraube komplementär zueinander ausgebildete kalottenförmige Lagerflächen aufweisen.

Weiter kann es sich als vorteilhaft erweisen, dass die Anlagescheibe und das proximale Ende oder der Kopf der Iliumsakralschraube in Umfangsrichtung einander drehhemmende Rastmittel aufweisen. Vorteilhaft ist auch eine möglich große Kontaktfläche zwischen Anlagescheibe und Ende oder Kopf der Schraube.
Zur weiteren Lagestabilisierung erweist es sich als vorteilhaft, dass die Anlagescheibe auf ihrer zur Iliumoberfläche weisenden Seite Verankerungsmittel aufweist in Form von hervorstehenden Zacken oder Stiften, welche beim Festziehen der Iliumsakralschraube in die Iliumoberfläche einzudringen vermögen.

Nach einem weiteren Gedanken von besonderer praktischer Bedeutung erweist es sich als vorteilhaft, dass die Anlagescheibe eine sich nach radial außen erweiternde durch Flanken begrenzte Radialöffnung aufweist. Bei entsprechender Ausbildung der Radialöffnung in Bezug auf den Außendurchmesser der Iliumsakralschraube kann die Anlagescheibe dann durch geringfügige elastische Aufweitung quer zur zweiten Längsrichtung auf den Schaft und insbesondere auf den eingeschnürten Bereich der Iliumsakralschraube aufgeklipst werden. Hierbei erweist es sich als vorteilhaft, wenn die Flanken verrundet sind und/oder verrundet in einen Außenumfang der Anlagescheibe übergehen, was das Aufklipsen erleichtert.

Die Anlagescheibe weist vorzugsweise eine maximale Dicke von 3,0 -6,0 mm auf, wobei die Dicke in radialer Richtung nach außen abnehmen kann.

Des weiteren erweist es sich als besonders vorteilhaft, wenn die Iliumsakralschraube ein zweigängiges Gewinde und in dem proximalen Gewindeabschnitt ein viergängiges Gewinde aufweist, welches dazu ausgebildet ist, über die volle Erstreckung des Hülsenelements in der ersten Längsrichtung in das Hülsenelement einzuschneiden.
Es wird weiter Schutz beansprucht für eine Sachgesamtheit umfassend einen erfindungsgemäßen Knochenanker und einer Polyaxialpedikelschraube mit einem Kugelkopfabschnitt und einem gabelförmigen Aufnahmeteil für den Kugelkopfabschnitt, wobei der Kugelkopfabschnitt mit einem distalen Gewindeabschnitt in eine axiale Öffnung in einem proximalen Endbereich der Iliumschraube einschraubbar ist. Wie oben schon ausgeführt, ermöglicht dies, dass der Knochenanker für Iliosakralosteosynthese außerdem mit einem oder mehreren angrenzenden Wirbelkörpern starr verbunden wird.

Weiter wird Schutz beansprucht für eine Sachgesamtheit umfassend einen erfindungsgemäßen Knochenanker und ein Werkzeug zum Eindrehen der Iliumschraube und eine Zielschablone, wobei das Werkzeug an einem proximalen Endbereich der Iliumschraube festlegbar ist und die Zielschablone an dem Werkzeug festlegbar ist, und wobei die Iliumschraube, das Werkzeug und die Zielschablone stets nur so aneinander festlegbar sind, dass eine Zielrichtung der Zielschablone immer durch die Durchgangsöffnung der Iliumschraube geht.

Hierbei erweist sich als vorteilhaft, dass die Zielschablone eine Zieleinrichtung für einen darin verschieblichen geraden Draht oder Stift aufweist und dass die Zieleinrichtung bezüglich einer bogenförmigen Führungsstrebe derart verstellbar ist, dass der gerade Draht oder Stift eine Schwenkebene beschreibt, wobei sich der Winkel des geraden Drahts oder Stifts zur ersten Längsrichtung der Iliumschraube ändert. Hierdurch kann ein gezielter Winkel oder Orientierung zwischen Iliumschraube und Iliumsakralschraube unter Verwendung der Zielschablone vorgegeben werden. Nach Einschrauben der Iliumschraube wird die Zielschablone hierfür an dem Werkzeug positioniert; es kann dann mittels der Zieleinrichtung die gewünschte Orientierung eingestellt werden und der Draht oder Stift in dieser gewünschten Orientierung durch das Ilium und durch die Durchgangsöffnung der Iliumschraube in das Sakrum hineingeführt werden. Der Draht oder Stift kann weiter als Führungshilfe beim Einschrauben der Iliumsakralschraube dienen.

Weiter wird Schutzbeansprucht für ein Verfahren mit den Merkmalen der Ansprüche 35 und 36.

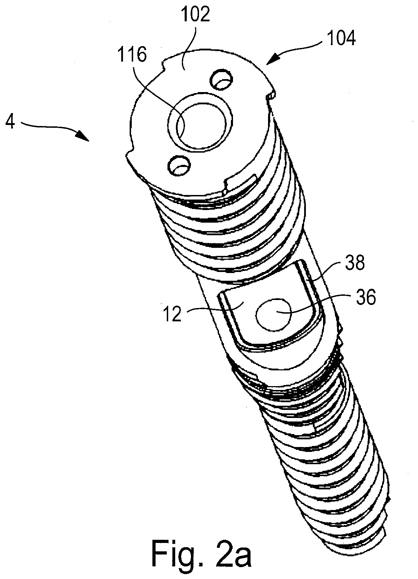

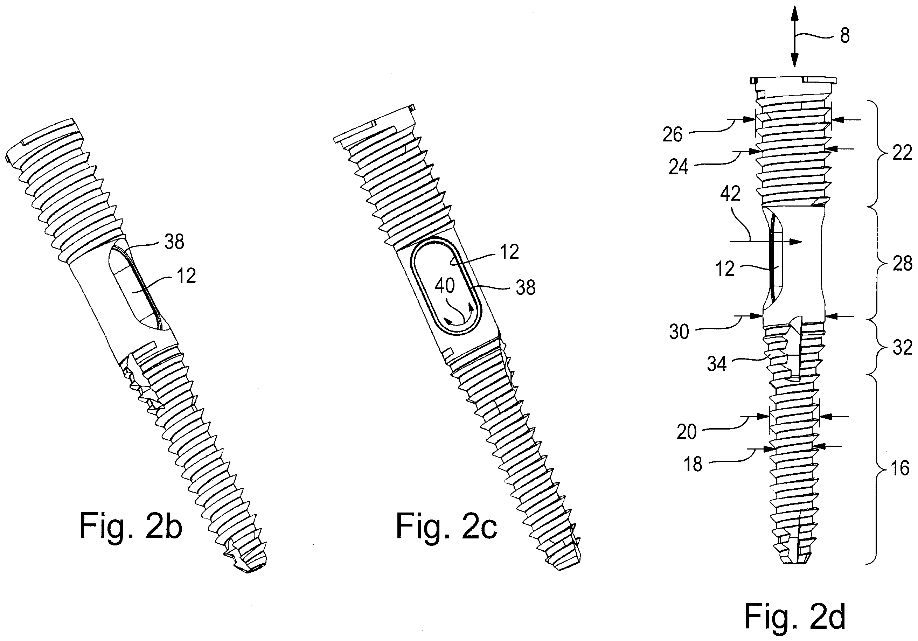

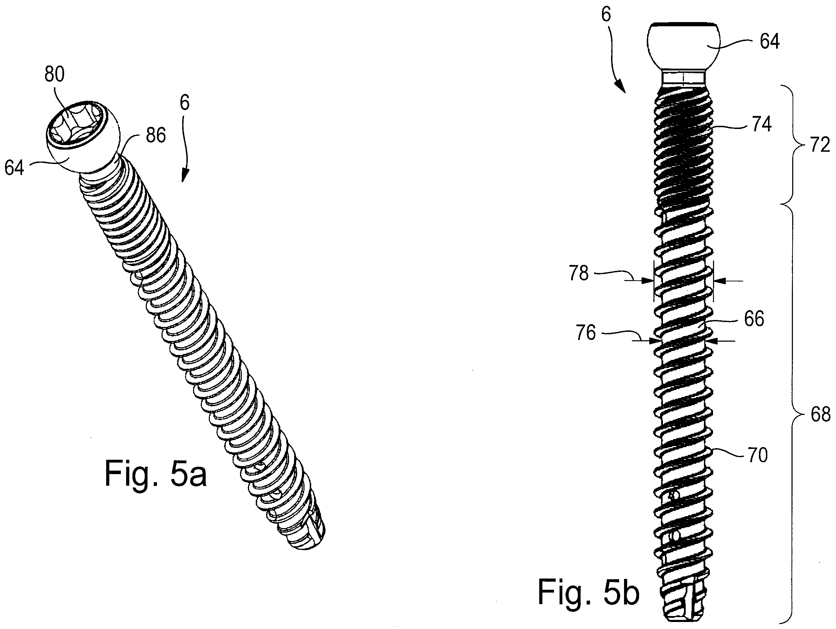

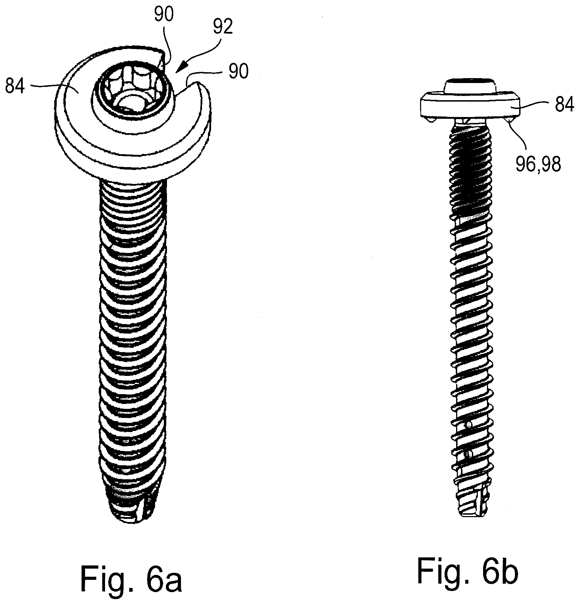

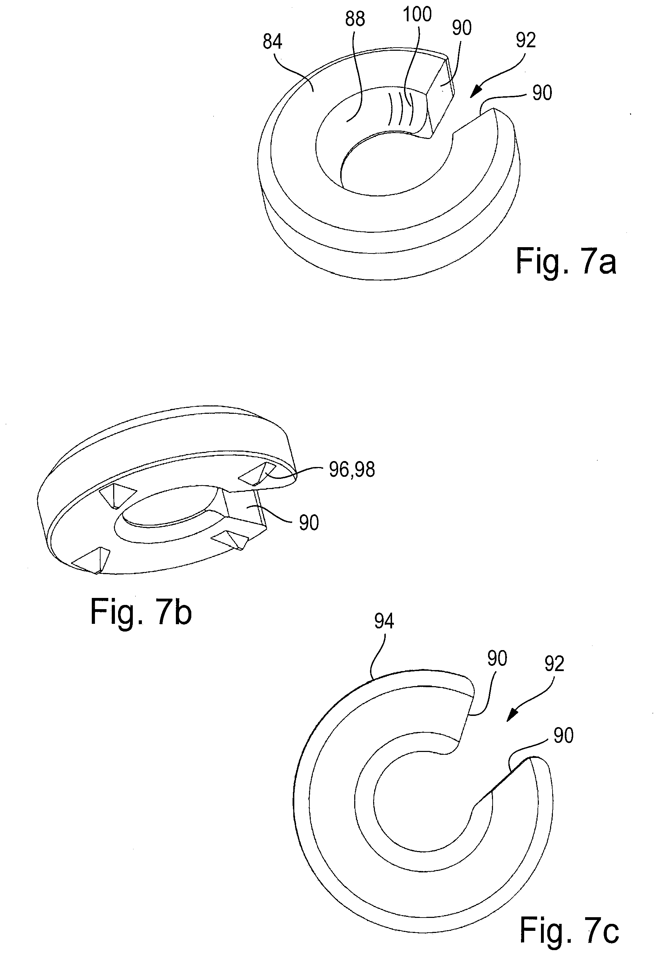

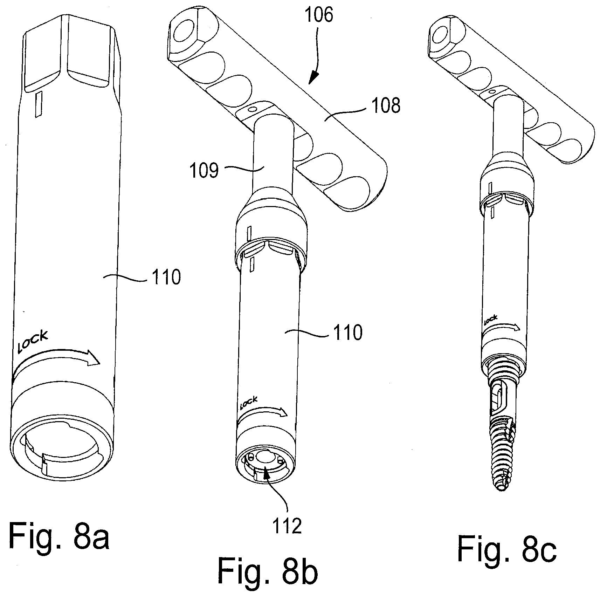

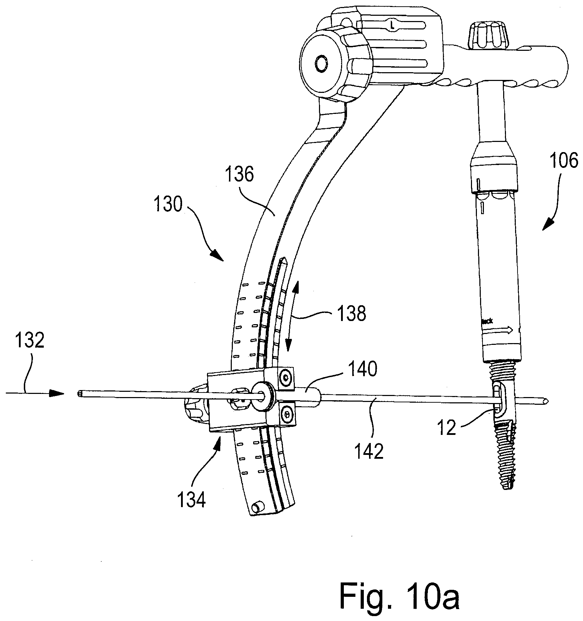

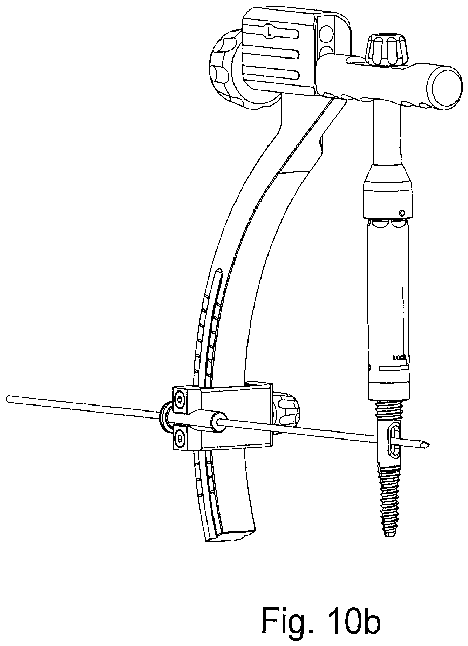

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Knochenankers. In der Zeichnung zeigt:

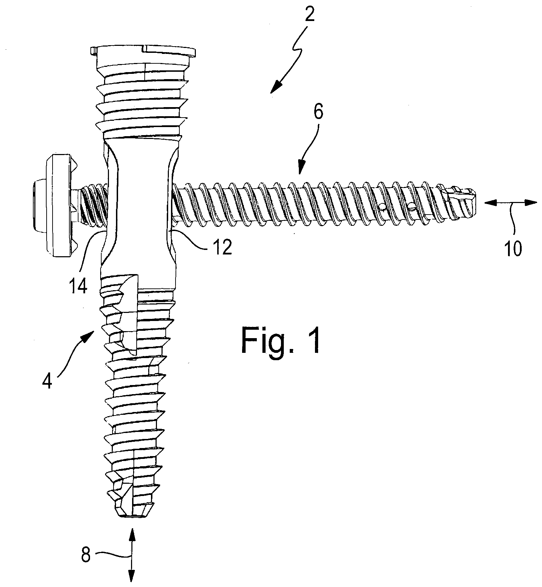

Figur 1 eine Seitenansicht eines erfindungsgemäßen
   Knochenankers für trianguläre Iliosakralostheosynthese, mit einer Iliumschraube und einer Iliumsakralschraube;
Figuren 2
   a)- d) verschiedene Ansichten der Iliumschraube nach Figur 1 ohne Hülsenelement;
Figuren 3
   a)- b) verschiedene Ansichten der Iliumschraube nach Figur 2, jedoch mit eingesetztem Hülsenelement;
Figuren 4
   a) - c) verschiedene Ansichten des Hülsenelements;
Figuren 5
   a), b) verschiedene Ansichten der Iliumsakralschraube nach Figur 1;
Figuren 6
   a), b) verschiedene Ansichten der Iliumsakralschraube nach Figur 5, jedoch mit einer Anlagescheibe;
Figuren 7
   a) - c) Ansichten der Anlagescheibe nach Figur 6;
Figuren 8
   a) - c) Ansichten eines Schraubendrehwerkzeugs mit Kopplungshülse und Drehgriff;
Figuren 9
   a) - c) einen in einer Pedikelschraube gelagerten Kugelkopfabschnitt; und Figuren 10
   a), b) Ansichten einer mit einer Iliumschraube zusammenwirkenden Zielschablone.

Figur 1 zeigt einen insgesamt mit dem Bezugszeichen 2 bezeichneten Knochenanker für trianguläre Iliosakralostheosynthese mit einer Iliumschraube 4 und einer Iliumsakralschraube 6. Die Iliumschraube 4 bildet eine erste Längsrichtung 8, und die Iliumsakralschraube 6 bildet eine zweite Längsrichtung 10, die hier nur beispielhaft orthogonal dargestellt sind. Die Iliumschraube 4 ist mit einer vorzugsweise langlochförmigen Durchgangsöffnung 12 ausgebildet, durch welche sich die Iliumsakralschraube 6 hindurcherstrecken kann. Bei der intendierten Anwendung der triangulären Iliosakralostheosynthese wird zuerst die Iliumschraube 4 in das Ilium eingeschraubt, und danach wird die Iliumsakralschraube 6 durch die Durchgangsöffnung 12 hindurch in das Sakrum eingeschraubt. Hierfür weist die Iliumschraube 4 - wie noch im Einzelnen beschrieben werden wird - in der Durchgangsöffnung 12 ein Hülsenelement 14 aus einem Polymermaterial auf, welches in die Durchgangsöffnung 12 eingesetzt ist. Im eingeschraubten Zustand der Iliumsakralschraube 6 schneiden deren Gewindegänge in das Hülsenelement 14 ein und verleihen so der Iliumsakralschraube 6 einen besseren Halt relativ zu der Iliumschraube 4.

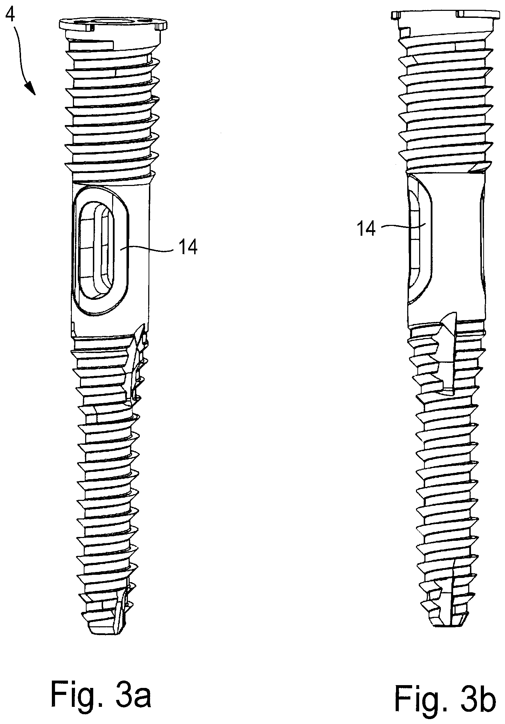

Die Iliumschraube 4 ist ohne dieses Hülsenelement 14 in den Figuren 2 a) bis d) dargestellt und mit eingesetztem Hülsenelement in den Figuren 3 a), b). Das Hülsenelement 14 ist außerdem in Figuren 4 a) - c) dargestellt.

Die Iliumschraube 4 weist einen distalen Gewindeabschnitt 16 mit einem distalen Kerndurchmesser 18 und einem distalen Nenndurchmesser 20 auf, sowie einen proximalen Gewindeabschnitt 22 mit einem proximalen Kerndurchmesser 24 und einem proximalen Nenndurchmesser 26 des Gewindes. Dazwischen ist ein gewindeloser Abschnitt 28 vorgesehen, in dem die vorzugsweise langlochförmige Durchgangsöffnung 12 quer und orthogonal zur ersten Längsrichtung 8 ausgebildet ist. Der proximale Nenndurchmesser 26 des proximalen Gewindeabschnitts 22 ist größer als ein Außendurchmesser des gewindelosen Abschnitts 28, und der Außendurchmesser 30 des gewindelosen Abschnitts 28 ist größer als der distale Nenndurchmesser 20. Ausgehend von dem gewindelosen Abschnitt 28, und zwar von einer Stelle distal zu der Durchgangsöffnung 12 nimmt der Außendurchmesser der Iliumschraube 4 in distaler Richtung bis zu dem distalen Gewindeabschnitt 16 ab. In diesem Bereich 32 abnehmenden Außendurchmessers ist ebenfalls ein Gewinde 34 vorgesehen, welches dann in das Gewinde des distalen Gewindeabschnitts 32 übergeht. Man erkennt ferner aus Figur 2 a) eine Öffnung, die sich als Längsmittelöffnung 36 durch die gesamte Iliumschraube 4 hindurcherstreckt und eine Kanülierung der Iliumschraube bildet.

Die Durchgangsöffnung 12 der Iliumschraube 4 ist mit einer Abstufung 38 ausgebildet. Diese Abstufung 38 bildet im beispielhaft dargestellten Fall eine in einer Umfangsrichtung 40 der Durchgangsöffnung 12 durchgehend erstreckte Stufe oder Anlagefläche für das Hülsenelement 14. Anstelle der Abstufung 38 könnten auch ein(e) oder mehrere Stege oder Ausnehmungen, insbesondere eine in der Umfangsrichtung 40 erstreckte Nut vorgesehen sein, mit der das Hülsenelement 14 abstützend und dabei in der Einpressrichtung 42 oder in Gegenrichtung einen Formschluss bildend zusammenwirken kann. Die Einpressrichtung 42 ist dabei orthogonal zur ersten Längsrichtung 8.

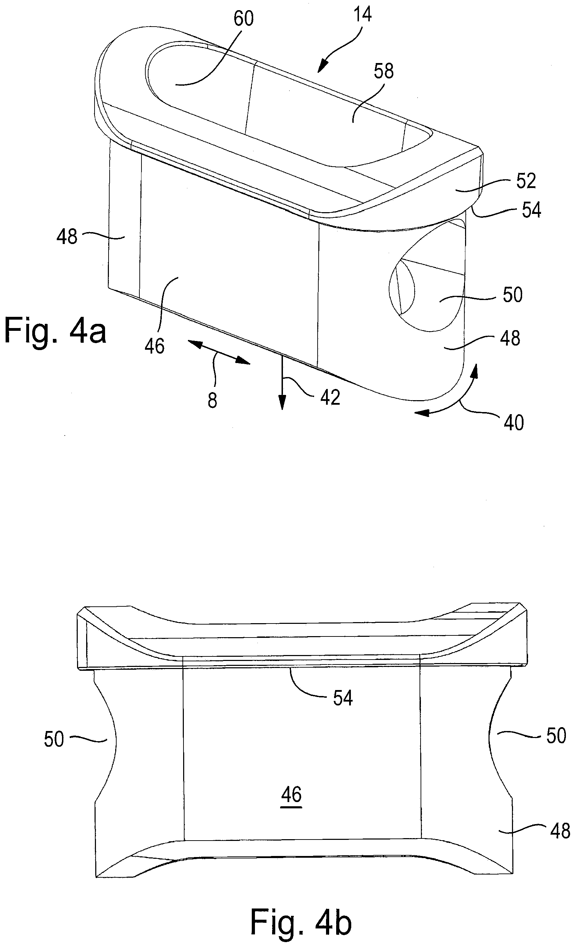

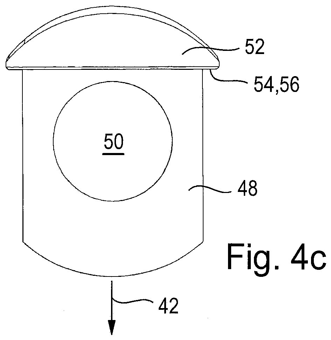

Das Hülsenelement 14 (vergl. Figuren 4 a) - c)) ist ebenfalls langlochförmig ausgebildet und umfasst eine in der Umfangsrichtung 40 geschlossene Wandung 46 mit zwei parallelen Wandungsabschnitten 48, die in der ersten Längsrichtung 8 erstreckt sind, und mit zwei diese Wandungsabschnitte 48 in der Umfangsrichtung 40 verbindende oval- oder halbrundförmige Wandungsabschnitte 48. In den Wandungsabschnitten 48 sind mit der Längsmittelöffnung 36 der Iliumschraube fluchtende Öffnungen 50 ausgebildet.

Das Hülsenelement 14 weist im beispielhaft dargestellten Fall einen quer zu seiner Einpressrichtung 42 vorstehenden Ansatz 52 auf, mittels dessen das Hülsenelement 14 einen formschlüssigen Hintergriff mit der Iliumschraube 4 ausbildet, wenn es in der Einpressrichtung 42 in die Durchgangsöffnung 12 eingesetzt wird. Im beispielhaft dargestellten Fall bildet der Ansatz 52 eine Abstufung 54, bei dem Hülsenelement 14, die in der Umfangsrichtung 40 durchgehend ausgebildet ist und einen durchgehenden Flansch 56 bei dem Hülsenelement 14 bildet. Mit diesem Flansch 56 liegt das Hülsenelement 14 im eingepressten Zustand gegen die Abstufung 38 an, die in der Durchstecköffnung 12 der Iliumschraube 4 ausgebildet ist. Hierdurch ist ein formschlüssiger Hintergriff in der Einpressrichtung 42 gebildet, das heißt, wenn weitere Kräfte in der Einpressrichtung 42 wirken, so kann das Hülsenelement 14 nicht in der Einpressrichtung 42 durch die Durchgangsöffnung 12 hindurchgedrückt werden, sondern es ist darin formschlüssig gehalten, indem die Abstufungen 38, 54 gegeneinander anliegen. Alternativ hierzu wäre auch ein Nut-/Federsystem denkbar oder eine sonstige Ausbildung eines in der Einpressrichtung 42 und in Gegenrichtung wirkenden formschlüssigen Ineinandergreifens von Hülsenelement 14 und Iliumschraube 4 denkbar und vorteilhaft. Wenn das Hülsenelement 14 in beiden Richtungen formschlüssig gehalten wäre, so würde es keine Vorzugsorientierung für das Einschrauben der Iliumsakralschraube 6 geben.

Insgesamt ist der Ansatz 52 an dem Hülsenelement 14 so ausgebildet, dass er im eingesetzten Zustand des Hülsenelements 14 flächenbündig und stufenlos in den Außenumfang des gewindelosen Abschnitts 28 übergeht. Der Ansatz 52 komplettiert gewissermaßen den Außenumfang des gewindelosen Abschnitts 28. Des Weiteren definiert das Hülsenelement 14 seinerseits eine langlochförmige Durchgangsöffnung mit zueinander parallelen Innenseiten 58 der Wandungsabschnitte 46 und mit gekrümmten Innenseiten 60 der Wandungsabschnitte 48.

Die Figuren 5 a), b) zeigen Ansichten der Iliumsakralschraube 6. Sie umfasst einen Kopf 64 und einen Schraubenschaft 66. Der Schraubenschaft 66 hat vorzugsweise einen distalen Gewindeabschnitt 68 mit einem beispielhaft zweigängigen Außengewinde 70 und einen proximalen Gewindeabschnitt 72 mit einem beispielhaft viergängigen Gewinde 74. Die Gewinde 70, 74 haben beispielhaft denselben Kern- und Nenndurchmesser. Beim Einschrauben der Iliumnsakralschraube 6 durch die Durchgangsöffnung 12 der Iliumschraube 4 hierdurch schneiden die Gewinde 70, 74 in die Wandungsabschnitte 46 und gegebenenfalls 48 des Hülsenelements 14 ein. Beim Einschrauben wird dabei auf das Hülsenelement 14 eine Zugkraft in der zweiten Längsrichtung 10 ausgeübt. Durch den formschlüssigen Hintergriff des Hülsenelements 14 mit der Iliumschraube 4 besteht jedoch nicht die Gefahr, dass das Hülsenelement 14 beim Einschrauben der Iliuimsakralschraube 6 aus der Durchgangsöffnung 12 der Iliumschraube 4 herausgezogen oder hindurchgezogen wird. Das Material des Hülsenelements 14 wird vielmehr durch das Einschneiden des Gewindes 70, 74 der Iliumsakralschraube 6 verdrängt und noch mehr in der Durchgangsöffnung 12 der Iliumschraube 4 verpresst. Die Iliumsakralschraube 6 weist im beispielhaft dargestellten Fall eine inbus- oder torxförmige Werkzeugansetzstelle 80 am Kopf 64 auf.

Die Iliumsakralschraube ist kanüliert ausgebildet. Auf diese Weise kann die Iliumsakralschraube über einen Führungsdraht bei der Implantation zugeführt werden.

Die Iliumsakralschraube ist im distalen Drittel mit seitlichen Perforierungen ausgebildet. Somit kann marktüblicher Knochenzement durch die Schraube appliziert werden. Dadurch kann eine zusätzliche Fixierung der Iliumsakralschraube im Sacralen Wirbelkörper erreicht werden.

Die Figuren 6 a), b) zeigen ferner eine Anlagescheibe 84, die radial geschnitten und derart ausgebildet ist, dass sie auf einen eingeschnürten Bereich 86 der Iliumsakralschraube 6 zwischen Kopf 64 und proximalem Gewindeabschnitt 72 aufschiebbar, insbesondere aufklipsbar ist. Die Anlagescheibe 84 ist in diesem Zustand zur zweiten Längsrichtung 10 neigbar, jedoch vorzugsweise unverlierbar an der Iliumsakralschraube 6 gehalten. Zwischen der Anlagescheibe 84 und der Iliumsakralschraube 6 sind komplementär zueinander ausgebildete kalottenförmige Lagerflächen 88 ausgebildet. Somit lässt sich die Anlagescheibe 84 auch geneigt zur zweiten Längsrichtung 10 gegen eine Außenfläche der Iliumoberfläche anlegen, wenn die Iliumsakralschraube 6 festgezogen wird. Die Anlagescheibe 84 weist vorzugsweise eine sich nach radial außen erweiternde und durch Flanken 90 begrenzte Radialöffnung 92 auf. Die Flanken 90 sind vorzugsweise verrundet oder gehen zumindest verrundet in einen Außenumfang 94 der Anlagescheibe 84 über. Auf ihrer im Ilium zugewandten Seite umfasst die Anlagescheibe 84 Verankerungsmittel 96 in Form von dort hervorstehenden Zacken oder Stiften 98, mit denen die Anlagescheibe 84 in die Knochenoberfläche des Ilium eindringen kann, wodurch die Verankerung weiter verbessert wird. Im Bereich der Lagerflächen 88 zwischen der Anlagescheibe 84 können nur schematisch angedeutete Rastmittel 100 vorgesehen sein.

Am proximalen Endbereich 102 der Iliumschraube 4 ist ebenfalls eine Werkzeugansetzstelle 104 ausgebildet, an der ein Drehwerkzeug 106 mit Handgriff 108, Drehspindel 109 und Hülse 110 drehfest anbringbar ist. Die Drehspindel 109 umfasst an ihrem distalen Ende komplementär zu dem proximalen Endbereich 102 ausgebildete Drehkopplungs- oder Eingriffsmittel 112. Handgriff 108, Drehspindel 109 und Hülse 110 sind drehfest mit der Iliumschraube 4 koppelbar, so dass die Iliumschraube 4 in das Ilium eingeschraubt werden kann. Das Drehwerkzeug 116 und dessen Komponenten sind so ausgebildet, dass sie nur in höchstens zwei um 180° voneinander verschiedenen Drehstellungen an der Iliumschraube festlegbar sind. Drehwerkzeug 116 gewissermaßen die Lage der Durchgangsöffnung 12 der Iliumschraube.

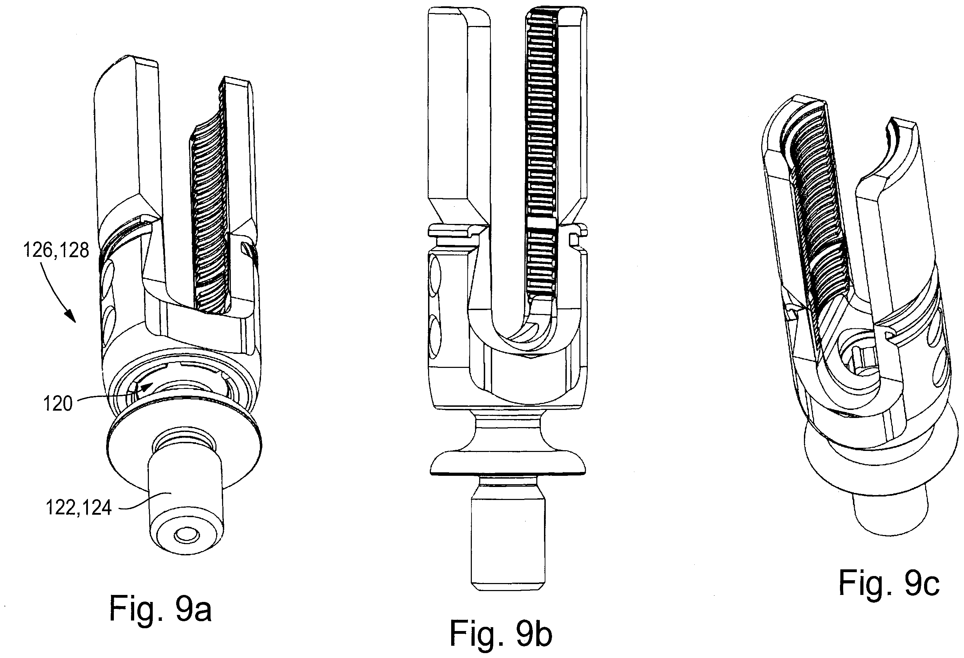

Die Figuren 9a-c) zeigen einen Kugelkopfabschnitt 120 mit einem Schaftabschnitt 122 mit einem distalen Gewindeabschnitt 124, der wiederum in die Gewindeöffnung 116 am proximalen Endbereich 102 der Iliumschraube 4 einschraubbar ist. Der Kugelkopfabschnitt 120 ist verschwenkbar aufgenommen in einem gabelförmigen Aufnahmeteil 126 einer Polyaxialpedikelschraube 128. In dem gabelförmigen Aufnahmeteil 126 ist in an sich bekannter Weise ein Stab fixierbar, mittels dessen die Pedikelschraube mit angrenzenden Pedikelschrauben verbindbar ist.

Schließlich zeigen Figuren 10a, b) eine Zielschablone 130, die an dem Drehwerkzeug 106 derart befestigbar ist, dass eine Zielrichtung 132 der Zielschablone 130 stets durch die Durchgangsöffnung 12 in der Iliumschraube 4 verläuft. Hierfür ist das Drehwerkzeug 106 nur in einer oder höchstens zwei bestimmten Orientierungen relativ zu der Durchgangsöffnung 12 an der Iliumschraube 4 ansetzbar. Dadurch, dass die Zielschablone 130 dann wiederum in vorbestimmter Orientierung an dem Drehwerkzeug 106 festlegbar ist, lässt sich diese Ausrichtung automatisch erreichen. Die Zielschablone 130 umfasst eine Zieleinrichtung 134, die in Bezug auf eine bogenförmige Führungsstrebe 136 in Richtung des Doppelpfeils 138 verstellbar ist. Die Zieleinrichtung 134 umfasst eine Aufnahme 140, in der ein Draht oder Stift 142 in der Längsrichtung 132 längsverschieblich geführt werden kann. Dieser Draht oder Stift 142 gibt dann bei geeigneter Ausrichtung der Zielschablone 130 die zweite Längsrichtung 10 für die Iliumsakralschraube 6 vor.

Des weiteren zeigt Figur 2 a) eine Gewindeöffnung 116, die sich in der ersten Längsrichtung, ausgehend vom proximalen Endbereich 102, erstreckt. In diese Gewindeöffnung kann eine Kappe oder ein Kugelkopfabschnitt eingeschraubt werden.

## Patentansprüche

1. Knochenanker (2) für trianguläre
Iliosakralostheosynthese mit einer Iliumschraube (4) mit einer ersten Längsrichtung (8) und einer Iliumsakralschraube mit einer zweiten Längsrichtung (10), wobei die Iliumschraube (4) in einem gewindelosen Abschnitt (28) eine vorzugsweise langlochförmige Durchgangsöffnung (12) quer zur ersten Längsrichtung (8) aufweist, wobei die Iliumsakralschraube (6) einen solchen gegenüber der Iliumschraube kleineren Außendurchmesser aufweist, dass sie durch diese Durchgangsöffnung (12) hindurchführbar ist und ausgehend vom Ilium durch die Iliumschraube (4) hindurch in das Sacrum einschraubbar ist, wobei die Iliumschraube (4) einen distalen Gewindeabschnitt (16) aufweist mit einem distalen Kerndurchmesser (18) und einem distalen Nenndurchmesser (20) des Gewindes und einen proximalen Gewindeabschnitt (22) mit einem proximalen Kerndurchmesser (24) und einem proximalen Nenndurchmesser (26) des Gewindes, und wobei der gewindelose Abschnitt (28) zwischen dem distalen (16) und dem proximalen Gewindeabschnitt (22) angeordnet ist, wobei der proximale Nenndurchmesser (26) größer ist als ein Außendurchmesser (30) des gewindelosen Abschnitts (28) und der Außendurchmesser (30) des gewindelosen Abschnitts (28) größer ist als der distale Nenndurchmesser (20), und wobei in die Durchgangsöffnung (12) ein der Form der Durchgangsöffnung komplementär entsprechendes Hülsenelement (14) aus einem Polymermaterial eingesetzt ist, welches in der Durchgangsöffnung (12) dann reibschlüssig und zusätzlich bezüglich mindestens einer Richtung formschlüssig gehalten ist, wobei eine Innenabmessung des Hülsenelements (14) orthogonal zur ersten Längsrichtung (8) geringer ist als ein Nenndurchmesser (78) eines Gewindes (70) der Iliumsakralschraube (6).

2. Knochenanker nach Anspruch 1, **dadurch gekennzeichnet, dass** das Hülsenelement (14) aus einem zwar formstabilen, jedoch geringfügig nachgiebig verformbaren, insbesondere elastisch nachgiebig verformbaren, Polymermaterial besteht, insbesondere aus Polyethylen, insbesondere aus ultra-hochmolekularem Polyethylen (UHMW PE), aus PEEK, PEAK, PEKK, PPSU oder PPS, gebildet ist, so dass es in Bezug auf die Durchgangsöffnung mit geringfügigem Übermaß ausgebildet und dann unter geringfügiger Verformung in die Durchgangsöffnung eingepresst werden kann.

3. Knochenanker nach Anspruch 1 oder 2, **dadurch gekennzeichnet, dass** eine Innenabmessung der Durchgangsöffnung (12) orthogonal zur ersten Längsrichtung (8) wenigstens 6,0 mm, insbesondere wenigstens 7,0 mm und weiter insbesondere höchstens 11,5 mm, insbesondere höchstens 10,5 mm, insbesondere höchstens 9,5 mm beträgt.

4. Knochenanker nach einem oder mehreren der vorstehenden Ansprüche, **dadurch gekennzeichnet, dass** eine Innenabmessung des Hülsenelements (14) orthogonal zur ersten Längsrichtung (8) geringer ist als ein Kerndurchmesser (76) eines Gewindes (74) der Iliumsakralschraube (6).

5. Knochenanker nach einem oder mehreren der vorstehenden Ansprüche, **dadurch gekennzeichnet, dass** ein Nenndurchmesser (78) eines Gewindes (74) der Iliumsakralschraube (6) wenigstens 6 mm und höchstens 9,5 mm, insbesondere höchstens 8,5 mm beträgt.

6. Knochenanker nach einem oder mehreren der vorstehenden Ansprüche, **dadurch gekennzeichnet, dass** das Hülsenelement (14) eine Wandstärke von wenigstens 1,0 mm, insbesondere von wenigstens 1,5 mm und von höchstens 3,5 mm, insbesondere von höchstens 3,0 mm, insbesondere von höchstens 2,5 mm aufweist.

7. Knochenanker nach einem oder mehreren der vorstehenden Ansprüche, **dadurch gekennzeichnet, dass** das Hülsenelement (14) wenigstens einen quer zu seiner Einpressrichtung (42) vorstehenden Ansatz (52) aufweist, der insbesondere eine Abstufung (54) an dem Hülsenelement (14) bildet, mittels dessen ein formschlüssiger Hintergriff des Hülsenelements (14) mit der Iliumschraube (4) erreicht wird.

8. Knochenanker nach Anspruch 7, **dadurch gekennzeichnet, dass** der Ansatz (52) oder die Abstufung (54) in einer Umfangsrichtung (40) des Hülsenelements (14) erstreckt ist und insbesondere einen in der Umfangsrichtung (40) unterbrochenen oder durchgehenden Flansch (56) an dem Hülsenelement (14) bildet.

9. Knochenanker nach einem oder mehreren der vorstehenden Ansprüche, **dadurch gekennzeichnet, dass** die Durchgangsöffnung (12) mit einer Ausnehmung oder Abstufung (38) ausgebildet ist, welche beim Einpressen des Hülsenelements (14) einen Hintergriff mit dem Hülsenelement ausbildet oder einen Endanschlag für das Hülsenelement, insbesondere für dessen Ansatz (52) oder Abstufung (54), bildet.

10. Knochenanker nach einem oder mehreren der vorstehenden Ansprüche, **dadurch gekennzeichnet, dass** der distale Nenndurchmesser (20) des distalen Gewindeabschnitts (16) der Iliumschraube (4) wenigstens 5,0 mm, insbesondere wenigstens 6,0 mm, insbesondere wenigstens 7,0 mm und höchstens 10,5 mm, insbesondere höchstens 9,5 mm, insbesondere höchstens 8,5 mm beträgt.

11. Knochenanker nach einem oder mehreren der vorstehenden Ansprüche, **dadurch gekennzeichnet, dass** der Außendurchmesser (30) des gewindelosen Abschnitts (28) im Bereich der Durchgangsöffnung (12) wenigstens 9,0 mm, insbesondere wenigstens 10,0 mm und höchstens 14,5 mm, insbesondere höchstens 13,5 mm, insbesondere höchstens 12,5 mm beträgt.

12. Knochenanker nach einem oder mehreren der vorstehenden Ansprüche, **dadurch gekennzeichnet, dass** der proximale Nenndurchmesser (26) des proximalen Gewindeabschnitts (22) der Iliumschraube (4) wenigstens 10,0 mm, insbesondere wenigstens 11 mm und höchstens 16,5 mm, insbesondere höchstens 15,5 mm, insbesondere höchstens 14,5 mm beträgt.

13. Knochenanker nach einem oder mehreren der vorstehenden Ansprüche, **dadurch gekennzeichnet, dass** ein Außendurchmesser (30) der Iliumschraube (4) ausgehend von dem gewindelosen Abschnitt (28) und von einer Stelle distal zu der Durchgangsöffnung (12) in distaler Richtung bis zu dem distalen Gewindeabschnitt (16) abnimmt und dass in dem Bereich (32) abnehmenden Außendurchmessers der Iliumschraube (4) ein weiteres Außengewinde (34) mit abnehmendem Nenndurchmesser ausgebildet ist.

14. Knochenanker nach einem oder mehreren der vorstehenden Ansprüche, **dadurch gekennzeichnet, dass** die Iliumsakralschraube (6) ein zweigängiges Gewinde und in dem proximalen Gewindeabschnitt (72) ein viergängiges Gewinde (74) aufweist, welches dazu ausgebildet ist, über die volle Erstreckung des Hülsenelements (14) in der ersten Längsrichtung (8) in das Hülsenelement (14) einzuschneiden.

15. Sachgesamtheit umfassend einen Knochenanker nach einem oder mehreren der vorstehenden Ansprüche und eine Polyaxialpedikelschraube mit einem Kugelkopfabschnitt und einem gabelförmigen Aufnahmeteil für den Kugelkopfabschnitt, wobei der Kugelkopfabschnitt mit einem distalen Gewindeabschnitt in eine axiale Öffnung (116) in einem proximalen Endbereich (102) der Iliumschraube (4) einschraubbar ist.

## Claims

1. Bone anchor (2) for triangular iliosacral osteosynthesis, comprising an ilium screw (4) having a first longitudinal direction (8) and an iliosacral screw having a second longitudinal direction (10), wherein the ilium screw (4), in a thread-free portion (28), has a preferably elongated-hole-like through-opening (12) transversely to the first longitudinal direction (8), wherein the iliosacral screw (6) has such a smaller outer diameter in relation to the ilium screw that the iliosacral screw can be guided through said through-opening (12) and can be screwed proceeding from the ilium into the sacrum, through the ilium screw (4), wherein the ilium screw (4) has a distal threaded portion (16) having a distal core diameter (18) and a nominal distal diameter (20) of the thread and a proximal threaded portion (22) having a proximal core diameter (24) and a proximal nominal diameter (26) of the thread, and wherein the thread-free portion (28) is arranged between the distal (16) and the proximal thread portion (22), wherein the proximal nominal diameter (26) is greater than an outer diameter (30) of the thread-free portion (28) and the outer diameter (30) of the thread-free portion (28) is greater than the distal nominal diameter (20), and wherein a sleeve element (14) having a shape complementary to the shape of the through-opening and made of a polymer material is inserted into the through-opening (12) and is then held in the through-opening (12) frictionally and additionally in a form-fitting manner relative to at least one direction, wherein the inner dimension of the sleeve element (14) orthogonally to the first longitudinal direction (8) is smaller than a nominal diameter (78) of a thread (70) of the iliosacral screw (6) .

2. Bone anchor according to claim 1, **characterized in that** the sleeve element (14) consists of a polymer material, in particular is made of polyethylene, in particular of ultra-high molecular weight polyethylene (UHMW PE), PEEK, PEAK, PEKK, PPSU or PPS, that is dimensionally stable, but slightly flexibly deformable, in particular elastically deformable, such that it can be slightly oversized with respect to the through-opening and can then be pressed with slight deformation into the through-opening.

3. Bone anchor according to claim 1 or 2, **characterized in that** an inner dimension of the through-opening (12) orthogonally to the first longitudinal direction (8) is at least 6.0 mm, in particular at least 7.0 mm and more particularly at most 11.5 mm, in particular at most 10.5 mm, in particular at most 9.5 mm.

4. Bone anchor according to one or more of the preceding claims, **characterized in that** an inner dimension of the sleeve element (14) that is orthogonal to the first longitudinal direction (8) is smaller than a core diameter (76) of a thread (74) of the iliosacral screw (6) .

5. Bone anchor according to one or more of the preceding claims, **characterized in that** a nominal diameter (78) of a thread (74) of the iliosacral screw (6) is at least 6 mm and at most 9.5 mm, in particular at most 8.5 mm.

6. Bone anchor according to one or more of the preceding claims, **characterized in that** the sleeve element (14) has a wall thickness of at least 1.0 mm, in particular of at least 1.5 mm and of at most 3.5 mm, in particular of at most 3.0 mm, in particular of at most 2.5 mm.

7. Bone anchor according to one or more of the preceding claims, **characterized in that** the sleeve element (14) has at least one projection (52) projecting transversely to its press-in direction (42), by means of which a form-fit engagement of the sleeve element (14) with the ilium screw (4) is achieved, the projection in particular forms a step (54) on the sleeve element (14).

8. Bone anchor according to claim 7, **characterized in that** the projection (52) or the step (54) is extended in a circumferential direction (40) of the sleeve element (14) and in particular forms a broken or continuous flange (56) on the sleeve element (14) in the circumferential direction (40).

9. Bone anchor according to one or more of the preceding claims, **characterized in that** the through-opening (12) is formed with a recess or step (38) which forms an engagement with the sleeve element or an end stop for the sleeve element during the pressing of the sleeve element (14), in particular for its projection (52) or step (54).

10. Bone anchor according to one or more of the preceding claims, **characterized in that** the distal nominal diameter (20) of the distal threaded portion (16) of the ilium screw (4) is at least 5.0 mm, in particular at least 6.0 mm, in particular at least 7.0 mm and is not more than 10.5 mm, in particular not more than 9.5 mm, in particular not more than 8.5 mm.

11. Bone anchor according to one or more of the preceding claims, **characterized in that** the outer diameter (30) of the thread-free portion (28) in the region of the through-opening (12) is at least 9.0 mm, in particular at least 10.0 mm and at most 14.5 mm, in particular at most 13.5 mm, in particular at most 12.5 mm.

12. Bone anchor according to one or more of the preceding claims, **characterized in that** the proximal nominal diameter (26) of the proximal threaded portion (22) of the ilium screw (4) is at least 10.0 mm, in particular at least 11 mm and is not more than 16.5 mm, in particular not more than 15.5 mm, in particular not more than 14.5 mm.

13. Bone anchor according to one or more of the preceding claims, **characterized in that** an outer diameter (30) of the ilium screw (4) decreases starting from the thread-free portion (28) and from a position distal to the through-opening (12) in the distal direction as far as the distal threaded portion (16), and that in the region (32) of the decreasing outside diameter of the ilium screw (4) a further outside thread (34) having a decreasing nominal diameter is formed.

14. Bone anchor according to one or more of the preceding claims, **characterized in that** the iliosacral screw (6) has a double-threaded thread and in the proximal threaded portion (72) a four-turn thread (74) which is designed to cut into the sleeve element (14) over the full extent of the sleeve element (14) in the first longitudinal direction (8).

15. Assembly comprising a bone anchor according to one or more of the preceding claims and a polyaxial pedicle screw having a ball head portion and a fork-shaped receiving part for the ball head portion, wherein the ball head portion can be screwed with a distal threaded portion into an axial opening (116) in a proximal end portion (102) of the ilium screw (4).

## Revendications

1. Ancre à os (2) pour l'osthéosynthèse sacro-iliaque triangulaire, comprenant une vis iliaque (4) présentant une première direction longitudinale (8) et une vis sacro-iliaque présentant une deuxième direction longitudinale (10), dans lequel la vis iliaque (4) comprend dans une portion non filetée (28) une ouverture de passage (12) de préférence en forme de trou oblong, transversale à la première direction longitudinale (8), dans lequel la vis sacro-iliaque (6) présentant un diamètre extérieur plus petit par rapport à la vis iliaque tel que l'on peut faire passer celle-ci à travers cette ouverture de passage (12) et qu'elle peut être vissée dans le sacrum à partir de l'ilium et en passant à travers la vis iliaque (4), dans lequel la vis iliaque (4) présente une portion filetée (16) distale ayant un diamètre de noyau (18) distal et un diamètre nominal (20) distal du filet ainsi qu'une portion filetée (22) proximale ayant un diamètre de noyau (24) proximal et un diamètre nominal (26) proximal du filet, et dans lequel la portion non filetée (28) est disposée entre la portion filetée (16) distale et la portion filetée (22) proximale, dans lequel le diamètre nominal (26) proximal est supérieur à un diamètre extérieur (30) de la portion non filetée (28) et le diamètre extérieur (30) de la portion non filetée (28) est supérieur au diamètre nominal (20) distal, et dans lequel un élément formant manchon (14) en une matière polymère qui correspond de manière complémentaire à la forme de l'ouverture de passage est inséré dans ladite ouverture de passage (12) et est alors maintenu par friction et en sus à engagement positif par rapport à au moins une direction à l'intérieur de l'ouverture de passage (12), dans lequel une dimension intérieure de l'élément formant manchon (14), orthogonale à la première direction longitudinale (8), est inférieure à un diamètre nominal (78) d'un filet (70) de la vis sacro-iliaque (6).

2. Ancre à os selon la revendication 1, **caractérisé par le fait que** ledit élément formant manchon (14) est constitué d'une matière polymère qui, certes, est stable en forme, mais légèrement déformable de manière souple, en particulier élastiquement déformable, en particulier de polyéthylène, en particulier de polyéthylène de masse molaire très élevée (UHMW PE), de PEEK, de PEAK, de PEKK, de PPSU ou de PPS de sorte qu'il peut être conçu avec une légère surmesure par rapport à l'ouverture de passage et peut donc être pressé dans l'ouverture de passage en étant déformé légèrement.

3. Ancre à os selon la revendication 1 ou 2, **caractérisé par le fait qu'**une dimension intérieure de l'ouverture de passage (12) orthogonale à la première direction longitudinale (8) fait au moins 6,0 mm, en particulier au moins 7,0 mm et encore en particulier 11,5 mm tout au plus, en particulier 10,5 mm tout au plus, en particulier 9,5 mm tout au plus.

4. Ancre à os selon une ou plusieurs des revendications précédentes, **caractérisé par le fait qu'**une dimension intérieure de l'élément formant manchon (14) orthogonale à la première direction longitudinale (8) est inférieure à un diamètre de noyau (76) d'un filet (74) de la vis sacro-iliaque (6).

5. Ancre à os selon une ou plusieurs des revendications précédentes, **caractérisé par le fait qu'**un diamètre nominal (78) d'un filet (74) de la vis sacro-iliaque (6) est de 6 mm au moins et de 9,5 mm tout au plus, en particulier de 8,5 mm tout au plus.

6. Ancre à os selon une ou plusieurs des revendications précédentes, **caractérisé par le fait que** l'élément formant manchon (14) présente une épaisseur de paroi de 1,0 mm au moins, en particulier de 1,5 mm au moins et de 3,5 mm tout au plus, en particulier de 3,0 mm tout au plus, en particulier de 2,5 mm tout au plus.

7. Ancre à os selon une ou plusieurs des revendications précédentes, **caractérisé par le fait que** ledit élément formant manchon (14) présente au moins un épaulement (52) qui fait saillie transversalement à sa direction d'introduction par pression (42) et qui forme en particulier un gradin (54) sur l'élément formant manchon (14), au moyen duquel on obtient que l'élément formant manchon (14) est en prise de derrière à engagement positif avec la vis iliaque (4).

8. Ancre à os selon la revendication 7, **caractérisé par le fait que** ledit épaulement (52) ou le gradin (54) s'étend dans une direction circonférentielle (40) de l'élément formant manchon (14) et, en particulier, forme une bride (56) interrompue ou continue dans la direction circonférentielle (40), sur l'élément formant manchon (14) .

9. Ancre à os selon une ou plusieurs des revendications précédentes, **caractérisé par le fait que** l'ouverture de passage (12) est réalisée avec un évidement ou gradin (38) qui, lorsque ledit élément formant manchon (14) est introduit par pression, forme une prise de derrière avec l'élément formant manchon (14) ou forme une butée de fin de course pour l'élément formant manchon, en particulier pour l'épaulement (52) ou le gradin (54) de celui-ci.

10. Ancre à os selon une ou plusieurs des revendications précédentes, **caractérisé par le fait que** le diamètre nominal (20) distal de la portion filetée (16) distale de la vis iliaque (4) est de 5,0 mm au moins, en particulier de 6,0 mm au moins, en particulier de 7,0 mm au moins et de 10,5 mm tout au plus, en particulier de 9,5 mm tout au plus, en particulier de 8,5 mm tout au plus.

11. Ancre à os selon une ou plusieurs des revendications précédentes, **caractérisé par le fait que** le diamètre extérieur (30) de la portion non filetée (28) au niveau de l'ouverture de passage (12) est de 9,0 mm au moins, en particulier de 10,0 mm au moins et de 14,5 mm tout au plus, en particulier de 13,5 mm tout au plus, en particulier de 12,5 mm tout au plus.

12. Ancre à os selon une ou plusieurs des revendications précédentes, **caractérisé par le fait que** le diamètre nominal (26) proximal de la portion filetée (22) proximale de la vis iliaque (4) est de 10,0 mm au moins, en particulier de 11 mm au moins et de 16,5 mm tout au plus, en particulier de 15,5 mm tout au plus, en particulier de 14,5 mm tout au plus.

13. Ancre à os selon une ou plusieurs des revendications précédentes, **caractérisé par le fait qu'**un diamètre extérieur (30) de la vis iliaque (4) diminue, à partir de la portion non filetée (28) et depuis un point distal à l'ouverture de passage (12), dans la direction distale jusqu'à la portion filetée (16) distale, et qu'un autre filet extérieur (34) ayant un diamètre nominal décroissant est réalisé dans la zone (32) de diamètre extérieur décroissant de la vis iliaque (4).

14. Ancre à os selon une ou plusieurs des revendications précédentes, **caractérisé par le fait que** la vis sacro-iliaque (6) présente un filet double et, dans la portion filetée (72) proximale, un filet quadruple (74) qui est conçu pour entailler dans l'élément formant manchon (14) sur l'ensemble de l'extension de l'élément formant manchon (14) dans la première direction longitudinale (8) .

15. Ensemble matériel comprenant un ancre à os selon une ou plusieurs des revendications précédentes et une vis pédiculaire polyaxiale ayant une portion à tête sphérique et une partie de réception en forme de fourche pour ladite portion à tête sphérique, dans lequel la portion à tête sphérique peut être vissée avec une portion filetée distale dans une ouverture axiale (116) située dans une zone d'extrémité proximale (102) de la vis iliaque (4).
